# EUROPEAN PATENT SPECIFICATION

(11) **EP 4 352 498 B1**
(45) Date of publication and mention of the grant of the patent: **22.04.2026**
(21) Application number: 23718067.4
(22) Date of filing: 17.03.2023
(51) Int. Cl.: G01N 27/414, G01N 33/00, G01N 27/333, G01N 33/46

(54) **PHYTO-ANALYSIS SENSOR**
PHYTOANALYSESENSOR
CAPTEUR DE PHYTO-ANALYSE

(30) Priority: 19.03.2022 IT 202200005294
(43) Date of publication of application: 17.04.2024
(73) Proprietor: Plantvoice Srl Sb, 39100 Bolzano (IT)
(72) Inventor: BECCATELLI, Tommaso, 43041 Bedonia (IT); BECCATELLI, Matteo, 43041 Bedonia (IT)
(74) Representative: Lunati & Mazzoni S.r.L.
(86) International application number: PCT/IT2023/050082
(87) International publication number: WO 2023/181082

(56) References cited:
- US-A1- 2004 256 644
- US-A1- 2021 132 000
- US-A1- 2022 085 309
- DIACCI CHIARA ET AL: "Diurnal in vivo xylem sap glucose and sucrose monitoring using implantable organic electrochemical transistor sensors", ISCIENCE, vol. 24, no. 1, 1 January 2021 (2021-01-01), US, pages 101966, XP055976517, ISSN: 2589-0042, DOI: 10.1016/j.isci.2020.101966
- ZHANG LIJUN ET AL: "Highly selective and sensitive sensor based on an organic electrochemical transistor for the detection of ascorbic acid", BIOSENSORS AND BIOELECTRONICS, vol. 100, 7 September 2017 (2017-09-07), pages 235 - 241, XP085272701, ISSN: 0956-5663, DOI: 10.1016/J.BIOS.2017.09.006
- LIMING BAI ET AL: "Biological Applications of Organic Electrochemical Transistors: Electrochemical Biosensors and Electrophysiology Recording", FRONTIERS IN CHEMISTRY, vol. 7, 7 May 2019 (2019-05-07), XP055603054, DOI: 10.3389/fchem.2019.00313
- NICOLA COPPEDÈ ET AL: "An in vivo biosensing, biomimetic electrochemical transistor with applications in plant science and precision farming", SCIENTIFIC REPORTS, vol. 7, no. 1, 23 November 2017 (2017-11-23), XP055457513, DOI: 10.1038/s41598-017-16217-4

## Description

### TECHNICAL FIELD OF INVENTION

The object of this invention is a phyto-analysis sensor of the type specified in the preamble to the first claim.

In particular, the sensor is configured to measure electrochemical parameters in the sap or other liquid fluids (hereinafter simply fluids) of a plant.

### DESCRIPTION OF THE KNOWN TECHNIQUE

As known, electrochemical analysis of the fluids of a plant are performed by analyzing the sap extracted from the plant itself. This solution only allows sample monitoring, thus not allowing continuous and real-time monitoring of the electrochemical parameters of the plant.

Therefore, in recent years, in order to monitor the physiological properties of plants, electrochemical sensors have been made consisting of metal electrodes to be inserted into the plant and used to carry out electrical impedance measurements and potentiometric or amperometric studies.

The known technique described above presents some important drawbacks.

These sensors are bio-incompatible with the plant which then rejects such electrodes. In addition, this bio-incompatibility leads the plant to progressively isolate the metal electrodes from the structure of the plant that retracts forming a cavity insulating the sensor from the plant around the metal electrode .

Another drawback lies in the fact that the known sensors, being able to highlight only variations in the electrical characteristics of the fluids of the plant, do not allow to perform a biochemical characterization of said fluids and therefore to concretely perform a phyto-analysis.

Another non-secondary drawback lies in the complexity of construction and installation and therefore in the high costs of the well-known phyto-analysis sensors.

Phyto-analysis sensors are known from e.g. Chiara Diacci, et al., "Diurnal in vivo xylem sap glucose and sucrose monitoring using implantable organic electrochemical transistor sensors", iScience, Volume 24, Issue 1, 2021, 101966, ISSN 2589-0042.

### SUMMARY OF THE INVENTION

In this situation, the technical task in support of the present invention is to design a phyto-analysis sensor able to substantially remedy at least part of the problems mentioned. As part of this technical task, it is an important purpose of this invention to conceive a phyto-analysis sensor which is biocompatible with the plant.

Another important purpose of the invention is to create a sensor that allows to perform a precise phyto-analysis in real-time, in a simple and minimally invasive way.

Another non-secondary purpose is to create an-easy-to-use phyto-analysis sensor with reduced costs.

The specified technical task and purposes are achieved by a phyto-analysis sensor as claimed in annex claim 1. Preferred embodiments of the invention are described in the dependent claims.

### BRIEF DESCRIPTION OF THE DRAWINGS

The characteristics and advantages of the invention are clarified below by the detailed description of the preferred embodiments of the invention, with reference to the drawings, in which:
**Fig. 1** shows a phyto-analysis sensor according to the invention;
**Fig. 2** illustrates the application of a phyto-analysis sensor;
**Fig. 3** presents a second phyto-analysis sensor according to the invention;
**Fig. 4** shows an assembly of the phyto-analysis sensor of Fig. 3;
**Fig. 5** is a section of Fig. 4;
**Fig. 6** introduces a further phyto-analysis sensor according to the invention;
**Fig. 7** schematizes a process for the construction of a phyto-analysis sensor according to the invention; and
**Fig. 8** shows a detection procedure performed by a phyto-analysis sensor according to the invention.

### DESCRIPTION OF PREFERRED EMBODIMENTS

In this document, measurements, values, shapes and geometric references (such as perpendicularity and parallelism), when associated with words such as "about" or other similar terms such as "almost" or "substantially", are to be understood as disregarding measurement errors or inaccuracies due to errors in production and/or manufacture and, above all, disregarding a slight deviation from the value, measurement, shape or geometric reference to which it is associated. For example, such terms, when associated with a value, preferably indicate a divergence of no more than 10% of that value.

Furthermore, when used, terms such as "first", "second", "upper", "lower", "main" and "secondary" do not necessarily identify an order, a relationship priority or relative position, but can simply be used to clearly distinguish the different components.

Unless otherwise indicated, "perpendicular", "transverse", "parallel" or "normal" or other terms of geometric positioning between geometric elements (e.g. axes, directions and lines) are to be understood as referring to their reciprocal geometric position between the corresponding projections. Such projections shall be defined on a single plane parallel to the plane or planes in which these geometric features are situated.

The measurements and data reported in this text are to be considered, unless otherwise indicated, as having been made in the ICAO International Standard Atmosphere (ISO 2533).

Unless otherwise specified, as is apparent from the following discussions, terms such as "processing", "computing", "determination", "calculation", or similar are considered to refer to the action and/or processes of a computer or similar electronic computing device that manipulates and/or transforms data represented as physical, such as electronic quantities of registers of a computer system and/or memories into, other data similarly represented as physical quantities within computer systems, records or other devices for storing, transmitting or displaying information.

With reference to the Figures, the phyto-analysis sensor according to the invention is globally indicated with the number **1.**

It is configured to measure electrochemical parameters in the sap or other fluids of a plant.

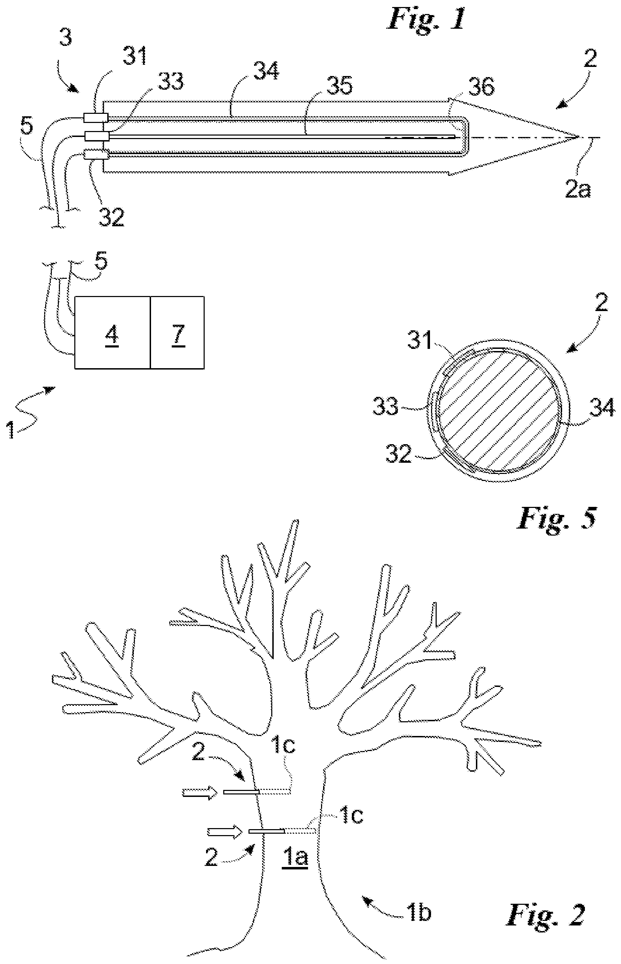

Phyto-analysis sensor 1 is configured to be at least partially (in detail preferably totally) inserted in in stem **1a** of plant **1b** as shown in Fig. 2. Appropriately it can be inserted into hole **1c** of stem **1b** almost counter-shaped to the below introduced rigid support 2. Stem 1a, as known to an expert technician in the field, identifies the supporting structure of a plant. It connects roots and leaves through the conductive tissues that have the function of transporting fluids (ie the arboreal fluid identifying the fluid and to be precise the liquid inside the plant and mostly including water and mineral salts) from the roots to the leaves, as well as the sap from these to the whole corm.

In detail, stem 1a is a woody stem.

The conductive tissues of the stem 1a may include wood or xylem (used for the conduction of water and mineral salts) and the bast or phloem (used for the conduction of lymph).

Hole 1c is made to make accessible to sensor 1 at least part and, in detail, all the conductive tissues of a section of plant 1b (in detail of stem 1a) precisely with the xylem and/or phloem. Phyto-analysis sensor 1 is configured to be inserted in stem 1a by coming into contact with the arboreal fluid and in detail with at least part and preferably all the conductive tissues of a section of the plant and, to be more precise, of stem 1a and in particular with the xylem / or phloem.

Phyto-analysis sensor 1 can include a sensitive device that can be inserted in plant 1b, to be precise, in stem 1a.

Phyto-analysis sensor 1 and, in detail, the sensitive device can include rigid support **2.** In detail, it includes only one rigid support.

Rigid support 2 is configured to fit into stem 1a. It is therefore suitable to go into direct contact with stem 1a.

Said support is defined as "rigid support" as it can be inserted into stem 1a without substantially modifying/deforming. It 2 is achievable in a material having a Brinell hardness substantially equal to at least 10 N/mm², in detail substantially between 10 N/mm² and 150 N/mm^{2,}and, more in detail, between 15 N/mm² and 100 N/mm^{2,} and, more in detail still, between 25 N/mm² and 50 N/mm² . This hardness being calculated according to the well-known Brinell scale is a scale of values derived from the test of hardness of materials with the Brinell method, devised by the Swedish metallurgist Johan August Brinell.

Rigid support 2 is configured to come into contact with stem 1a and absorb tree fluid from stem 1a. It is then configured to be inserted into stem 1a coming into contact with at least part of the conductive tissues of a section of stem 1a and, in particular, with the xylem/or phloem. Preferably, rigid support 2 identifies the only sensor element configured to absorb the arboreal fluid.

To this end, rigid support 2 is achievable in a material with a porosity substantially equal to at least 40% so as to allow the fluid to be absorbed by rigid support 2. Preferably, the porosity of rigid support 2 can be almost between 50% and 90%,, in detail, between 50% and 75% and ,more in detail, between 50% and 60%.

The porosity, typical of a structure with small gaps i.e. gaps and pores that can be filled with a fluid (i.e. the arboreal fluid), can be calculated as the ratio between the volume of the voids and the total volume of an absorber sample 2.

Preferably, rigid support 2 is made of a material having said Brinell hardness and said porosity.

Suitably, rigid support 2 is made of electrically insulating material.

Rigid support 2 can be made of hygroscopic material (i.e. with the ability to absorb moisture), preferably biocompatible.

It can be made of hydrophilic material (ie with the ability to absorb liquids such as those of stem 1a) preferably biocompatible.

Advantageously, rigid support 2 can be made of hydrophilic and hygroscopic material, preferably biocompatible. It can be made of natural material suitably in wood and, more appropriately, in bamboo or rattan.

It can define longitudinal axis **2a,** i.e. an axis of preferred development. Preferably, longitudinal axis 2a identifies the direction of the insertion of support 2 and then of phyto-analysis sensor 1 in stem 1a.

Rigid support 2 may also define a section normal to longitudinal axis 2a having an appropriate cylindrical shape, preferably with a diameter substantially between 5 mm and 15 mm. Alternatively, the section can be poly-lateral (as quadrilateral as rectangular or square), preferably with a top encumbrance of substantially between 5 mm and 15mm. The term rigid support section 2 identifies an area normal to axis 2a of the same rigid support.

Appropriately, it may have along longitudinal axis 2a said constant section or, in some cases, have at least one tapered section so as to facilitate the introduction into stem 1b. In particular, the rigid support can define, along axis 2a, a first end with tapered section and a second non-tapered section.

The section of rigid support 2 may have an extension of almost 0,1 cm² and, in detail, between 0,1 cm² and 5 cm²,in more detail, between 0,5 cm² and 3 cm². It can be substantially equal to 1.5 cm² or 2.0 cm².

Phyto-analysis sensor 1 and, in detail, the sensitive device may include at least one OECT 3 (in detail, only one) configured to perform an electrical characterization of the arboreal fluid and therefore allow an analysis of the fluid according to said electrical characterization.

In particular, OECT 3 is configured to perform electrical characterization of the arboreal fluid by detecting a current variation in the OECT itself. It therefore allows an analysis of the fluid in function of this variation of current.

OECT 3 can be deposited on rigid support 2 for example for molding. It should be noted that the OECT has a particularly reduced thickness and therefore almost does not alter the shape of the support allowing the introduction of the sensor in hole 1c almost counter shaped to rigid support 2. To this end, the rigid support may comprise active sector **2b** (e.g. identifiable in grooves) defining hollowed portions of support 2 on which OECT 3 is deposited, which therefore does not protrude from support 2.

OECT 3 is an organic electrochemical transistor. It may comprise a drain electrode **31;** a source electrode **32;** a gate electrode **33** sensitive to at least one solute in that arboreal fluid.

It should be noted that electrodes 31, 32, 33 are not in contact with each other.

Drain electrode 31 and source electrode 32 can be known metal electrodes bound to rigid support 2, for example, by gluing or interlocking. In some cases they can be made by deposit (such as molding) of electrically conductive material (such as a polymer and/or an electrically conductive paint) on rigid support 2. The conductive paint may include an electrically conductive powder (e.g. metallic such as copper, silver and/or gold) or a polymer dispersion, e.g. comprising at least one conductive homopolymer. In the case of metal powder, the paint may include powders of at least one first and one second metal having a standard reduction potential greater than the first metal, suitably almost 1V. For example, paint can have a silver powder content of substantially between 15% and 20% and a copper powder content of substantially between 3% and 5%.

Drain electrode 31 and source electrode 32 can be integral to rigid support 2 and, in detail, bound to the same surface of rigid support 2.

In a first non-limiting embodiment, electrodes 31 and 32 can be at the same end and, for example, at the second end (Fig. 1).

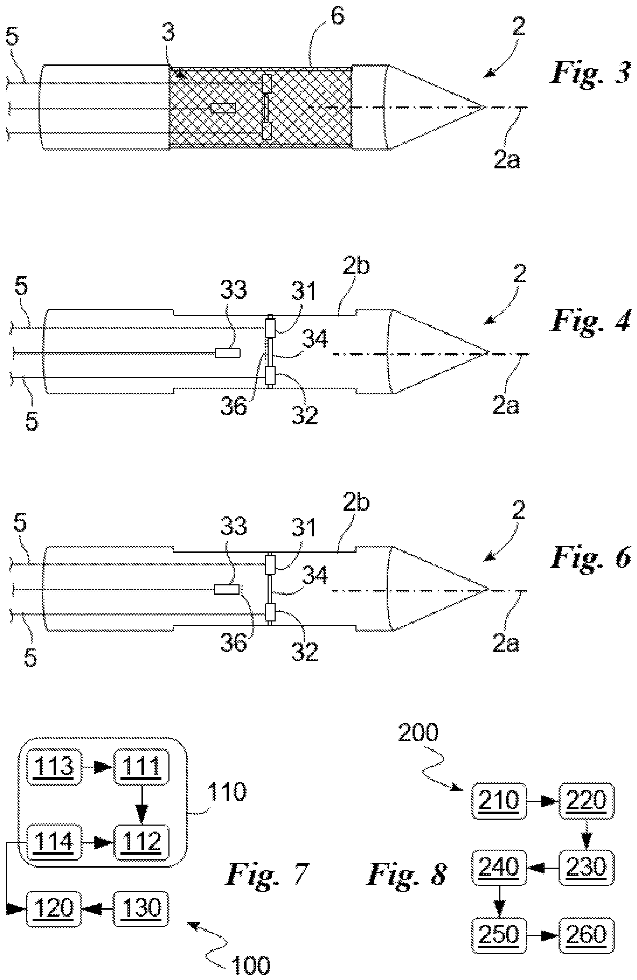

In a second non-limiting embodiment (Figs. 3-6), drain electrode 31 and source electrode 32 can be distal from the ends so as to be appropriately placed near the central section of stem 1a. For example, they can have a distance from each end substantially between 10% and 50%, in detail, between 25% and 50% and, more precisely, between 40% and 50% of the length of the rigid support calculated along the longitudinal axis 2a. Preferably according to this embodiment electrodes 31 and 32 are placed on substantially the same section of support 2.

At least in the second **embodiment,** the distance between electrodes 31 and 32 may be substantially less than 10 mm, in detail, less than 5 mm and appropriately, substantially between 1 mm and 3 mm (e.g. 2 mm). Said distance identifies a measurement carried out along the outer surface of rigid support 2.

Gate electrode 33 is configured to allow OECT 3 and, in detail, phyto-analysis sensor 1 to detect the content and, more precisely, the concentration of at least one solute in the arboreal fluid such as sodium, calcium and potassium. It may include an additive electrically sensitive to said solute described below and covering at least part of electrode 33.

Gate electrode 33 can be a well-known metal electrode bound to rigid support 2,for example, by gluing or interlocking. In some cases it can be made by deposit (such as molding) of electrically conductive material (such as a polymer and / or said electrically conductive paint) on rigid support 2.

Gate electrode 33 can be integral to rigid support 2 and, in detail, bound to a surface of support 2 distinct, or preferably coincident, with that of constraint of electrodes 31 and 32 (Fig. 1).

It can be placed between electrodes 31 and 32 , not in contact with them.

In said first **embodiment,** all electrodes 31, 32 and 33 can be placed at the same end (for example the second end as shown in Fig. 1).

In said second **embodiment,** electrode gate 33 is not at one end. For example, it can have a distance from each end substantially between 10% and 50% and, in detail, between 25% and 50% and to be more precise, between 40% and 50% of the length of support 2.

Preferably, in the **second embodiment** gate electrode 33 has a different distance from the ends from that of drain 31 and source 32 electrodes. Then electrodes 31 and 32 can be placed at a first section of rigid support 2 and electrode gate 33 at a second section of rigid support 2 distinct from the first section.

The distance between the first and second section, calculated along axis 2a, may be substantially less than 10 mm, in detail, less tham 5 mm and, appropriately between 1 mm and 3 mm (e.g. 2 mm).

The distance between the first and second section can be substantially equal to the distance between drain 31 and source 32 electrodes.

OECT 3 may include a first channel (**34)** connecting drain electrode 31 to source 32 and integral to rigid support 2 so as to be wet by the arboreal fluid, preferably arboreal, absorbed by rigid support 2.

The first channel (34) is achievable on the outer surface of rigid support 2 and not does not protrude from it. The first channel (34) can identify a groove of rigid support 2 which collects the preferably arboreal fluid; Consequently, the first channel (34) does not protrude from rigid support 2 which therefore defines the external profile of at least the portion of sensor 1 to be inserted into stem 1a.

It may not be in contact with gate electrode 33.

The first channel (34) can be electrically conductive. It can be realized by deposit (as molding) of electrically conductive material (such as a polymer and/or said electrically conductive paint) on rigid support 2.

The first channel (34) can define a first development trajectory and a first thickness normal to said development trajectory and parallel to the surface on which it is made.

The first development trajectory and the first channel (34) can extend for at least 50%, in detail, 70% and, more in detail, 90% of the length of rigid support 2 calculated along longitudinal axis 2a. Preferably, they extend almost all along rigid support 2.

The first channel (34) can also define an open projection profile in a plane perpendicular to longitudinal axis 2a. For example, in the first **embodiment,** the first development trajectory of the first channel (34) can be substantially a "U" as shown in Fig. 1; in the second **embodiment,** the first channel (34) can extend along part of the perimeter of the first section and thus identify an arch suitably lying on that first section.

Alternatively, the first channel (34) can define a closed projection profile on a plane perpendicular to longitudinal axis 2a. Therefore, the projection of the first channel (34) on that plane normal to the longitudinal axis 2a extends over the entire perimeter of a section of support 2 normal to axis 2a. For example, in the case of the second **embodiment,** said first channel (34) can identify a helix with multiple windings on support 2 or extend throughout the perimeter of the first section by defining, for example, a circle /circumference passing through and joining drain 31 and source 32 electrodes(Fig. 5).

Said projection of the first channel (34) is carried out along axis 2a.

It should be noted that gate electrode 33 is configured to interact through rigid support 2 (in detail, through at least part of the arboreal fluid absorbed by support 2) with the first channel (34) determining a variation of current in the first channel (34) and therefore between drain electrode 31 and source electrode 32 as a function of the solute content in the fluid.

In particular, gate 33 electrode is configured to interact with the first channel (34) directly (i.e. without additional elements) as shown in Figs. 3-6.

Alternatively, it is configured to interact with the first channel (34) indirectly (Fig. 1). Thus, OECT 3 may comprise a second channel (**35)** connected to gate electrode 33 and integral to rigid support 2 so as to be wet by the arboreal fluid, preferably arboreal, absorbed by the same support 2 allowing gate electrode 33 to interact through the second channel (35) with the first channel (34) by varying the current in said first channel (34) according to the content of said solute in said fluid.

The second channel (35) is achievable on the outer surface of support 2. It can be achieved in a groove of support 2 of collection of the fluid preferably arboreal, consequently, the second channel (35) does not protrude from rigid support 2 which therefore defines the external profile of the portion of sensor 1 to be inserted into stem 1a.

The second channel (35) can be non-contact with the first channel (34) and with electrodes 31 and 32.

It can be electrically conductive, in detail, sensitive to solute ions and preferably supplementary solute. It can be made by deposit (as molding) of electrically conductive material (such as a polymer and/or said electrically conductive paint) on rigid support 2. The second channel (35) can define a second development trajectory and a second thickness normal to that second development trajectory and parallel to the surface on which it is made.

The second development trajectory and then the second channel (35) can extend by at least 50%, in detail, 70% and, more in detail, 90% of the length of rigid support 2 calculated along longitudinal axis 2a. Preferably they extend almost all along rigid support 2.

The second developmental trajectory can be substantially parallel to the first trajectory. For example, as shown in Fig. 1 relating to the first **embodiment,** the second channel (35) may be surrounded by the first channel (34) and parallel to the linear sectors of said first channel (34).

It should be noted that at least in the second **embodiment** OECT 3 may be without second channel (35).

OECT 3 may include an additive **36** electrically sensitive to this solute, i.e. configured to vary its electrical characteristics (i.e. channel 34 or 35 preferably of the first channel (34)) as a function of the solute and, in detail, the concentration of solute in the arboreal fluid absorbed by support 2.

Additive 36 can include a polymer with a molecular fingerprint affine to the solute and therefore able to modify the current (in detail, the passage and, more precisely, the intensity of the current) in the first channel (34) (therefore the intensity of the current detected by the phyto-analysis sensor 1) depending on the presence of solute in the fluid absorbed by the support 2. Preferably, additive 36 is called molecularly imprinted polymer (MIP). The molecularly imprinted polymer and additive 36 can be affined to the solute. MIP identifies an additive obtained through molecular imprinting which creates a polymer matrix with cavities having affinity for a chosen "mold" molecule. This "mold" molecule is the solute to be detected.

Additive 36 can be configured to come into contact with the arboreal fluid absorbed by rigid support 2 and then interpose, at least in part, between a portion of OECT and said fluid.

It may be interposed at least partially and, in detail, fully between rigid support 2 and at least one portion and, in detail, the totality of at least one channel 34 or 35; and/or additive 36 may stand at least partially and, in detail, fully between rigid support 2 and gate electrode 33.

Additive 36 can be applied to at least part of channel 34 or 35 so as to stand between channel and electrode. Preferably additive 36 can stand at least in part and, in detail, totally between the first channel (34) and electrode gate 33.

In particular, it can be applied on at least part of the first channel (34) (Figs. 3-4) and, in detail, on the surface of the first channel (34) proximal and facing gate 33. Additionally or alternatively, additive 36 can be applied to at least part of the gate 33 electrode (Fig. 6) and preferably between gate 33 electrode and the first channel (34).

Additive 36 is applicable to channel 34 and/or 35 and/or gate 33 through integration and/or because it defines a coating of at least part of that component. In the first case (integration) additive 36 is an ingredient to be added (for example by mixing with electrically conductive material) in the formation of at least part of gate electrode 33 and/or channel 34 and/or 35; in the second case it may be a coating of at least part of electrode gate 33 and/or channel 34 and/or 35.

Additive 36 preferably does not protrude from rigid support 2. For example, if placed in channel 34 or 35 it fills it only partially.

Sensor 1 may include an OECT 3 operating control board **4** and at least one board **5** connector 4 to OECT 3.

Each connector 5 defines a data transfer between board 4 and OECT 3 and in detail between board 4 and each electrode.

Connector 5 can define a wireless or preferably wired connection (i.e. cables suitably equipped with electrically insulating coating).

Board 4 can be integrated into Stand 2.

Board 4 is configured to determine the presence and, in detail, the content and therefore the concentration of solute in the arboreal fluid as a function of the current variation in the first channel (34) (ie between electrodes 31 and 32) determined by the interaction of the electrode gate 33 and then of second channel 34 with first channel 34.

Board 4 may include an interface.

Said interface can be output and then include a screen or other device configured to allow an operator to view the outcome of the analysis. Additionally or alternatively, it can be input and therefore include a keyboard or other device configured to allow an operator to control the phyto-analysis sensor 1.

Board 4 can include a phyto-analysis database associating the value of a physiological parameter of plant 1b (such as the degree of hydration, correctness of feeding) to the content/concentration of one or more solutes.

It may also include a parameter database including rigid support 2 characteristics such as porosity, permeability or other characteristics described above.

Board 4 may include a storage memory for said databases and/or data acquired by the phyto-analysis sensor 1.

It can be configured to define a current transfer in OECT 3 and appropriately detect the presence and, in detail, the concentration of solute in the arboreal fluid, suitably arboreal, as a function of a variation of current in OECT 3 in detail between drain electrode 31 and source electrode 32.

In detail, board 4 is configured to apply potential, appropriately independently, to one or more electrodes by defining a passage of currents between said electrodes and therefore in OECT 3.

It can then define one or more acquisition configurations of a current between a pair of electrodes in each of which a potential difference is defined between two electrodes and, in detail, between drain electrode 31 and source electrode 32 appropriately for an uptime. The uptime can be substantially at least 30" (30 seconds), in detail, between 30" and 600", and in more detail, between 1 minute and 5 minutes and, even more in detail still, between 1 minute and 3 minutes. It is preferably almost equal to 2 minutes.

Uptime can be the same for various configurations.

The potential difference can be defined with drain electrode 31 discharge (at zero potential) and, in detail, only the source electrode 32 positively charged.

Board 4 can define a first acquisition configuration by defining a first potential difference between drain electrode 31 and source electrode 32.

The first potential difference can be almost less than 1 V, in detail, less than 0.5 V, and, in more detail, substantially between 0.3 V and 0.05 V. It can be substantially equal to 0.1 V.

In such configuration, gate electrode 33 is discharged.

Board 4 can define a second acquisition configuration by defining a second potential difference between drain electrode 31 and source electrode 32 and a charge potential of gate electrode 33.

The second potential difference can be almost less than 1 V, in detail, less than 0.5 V, and, more in detail, substantially between 0.3 V and 0.05 V. It can be substantially equal to 0.1 V.

The second potential difference can be equal to the first.

The charge potential can be positive.

It can be substantially greater than the second potential difference.

The charge potential can be substantially at least 0.2 V, in detail, substantially between 0.2 V and 5 V, and, more in detail, between 0.5 V and 2 V. It can be substantially between 0.8 V and 1 V.

It should be noted that in this configuration, since the gate electrode 33 is charged and drain electrode 31 is discharged, there is a potential difference between them.

Board 4 may include means of connection configured to connect phyto-analysis sensor 1 (in detail, Board 4) to an external unit such as a computer, tablet or smartphone.

The phyto-analysis sensor 1 and in detail the sensitive device can include sheath 6 (for simplicity, shown only in Fig. 3) of protection of at least part of the OECT.

It can be configured to protect at least electrodes 31, 32 and 33, the first channel (34) and, if present, the second channel (35). It can be configured to protect the entire OECT 3 which is then enclosed between sheath 6 and rigid support 2.

Sheath 6 covers at least part of rigid support 2 and at least part of the OECT enclosing at least electrodes 31, 32 and 33 and the first channel (34) between rigid support 2 and sheath 6. Therefore, in use (i.e. when at least rigid support 2 and OECT 3 are inserted into stem 1a), sheath 6 stands between stem 1a at least rigid support 2 and OECT 3 so as to avoid direct contact between OECT 3 and stem 1a.

Sheath 6 may not protrude from rigid support 2. As a result, active sector 2b accommodates OECT 3 and sheath 6, which therefore do not protrude from support 2. Suitably sheath 6 is made of electrically insulating material.

Sheath 6 can be made of hydrophilic material so as to absorb and therefore be penetrated by the arboreal fluid of plant 1b and, preferably, biocompatible.

Sheath 6 can be integral to rigid support 2.

Sheath 6 can be made of stretch material so as to be elastically deformed when wrapping support 2 and then tend to compress on it by constraining.

It can be made of natural material suitably in fabric, preferably stretch.

Sheath 6 can be made of perforated material. The term "perforated" means that sheath 6 can substantially include a perforated membrane, for example a retina or gauze or other, including through holes that facilitate the passage of the fluid that passes both through the material constituting sheath 6 and through said through holes. Phyto-analysis sensor 1 can include a phyto-analysis sensor power supply 7 such as a battery or connector to an external power supply network.

Power supply 7 can be configured to power sensor 1 according to board 4.

The invention includes a new construction **procedure 100** of a phyto-analysis sensor 1 in accordance with what is described above in structural terms.

**Construction procedure** 100 can be carried out according to Fig. 7.

**Construction procedure** 100 may comprise an implementation step **110** of OECT 3 on rigid support 2 appropriately corresponding to active sector 2b.

In said phase 110 it is possible to have the deposit (molding) of at least one electrically conductive material on support 2 creating the first channel (34),the second channel (35), if present, and appropriately electrodes 31, 32 and 33. In detail, **construction phase** 110 can include a first subphase of deposit 111 on the active sector 2b of a first electrically conductive material creating the first channel (34) and if present, the second channel (35). It may also include a second subphase of deposit 112 on the active sector 2b of a second electrically conductive material creating electrodes 31, 32, 33.

In said subphases 111 and 112 the deposit of said materials may be carried out by molding, in detail, be spraying, serigraphy, screen printing or pad printing.

In the first subphase 111 a first adhesive mask can be used reproducing in negative the first channel (34) and, if present, the second channel (35).

In the first subphase 111 the deposit can be made by heating rigid support 2 to a temperature substantially equal to said heating temperature.

In the second subphase 112 of deposit it can be used using a second adhesive mask reproducing the electrodes in negative.

The second material defining electrodes 31 and 32 is deposited on part of the first channel (34) and on part of support 2. The one defining gate electrode 33 is deposited on part of support 2 and, if present, on part of the second channel (35).

The first material may be different or preferably the same as the second material.

The first material can be the polymer paint described above. Preferably, it is sensitive to solute and/or supplementary solute ions and, in detail, can be an electrically conductive polymer such as paint, preferably with emulsion particles of a conductive polymer such as polypyrrole, polyaniline or PEDOT-PSS.

The second material can be a metallic paint comprising metal powders of, for example, copper, silver and/or gold as described above.

In each deposit subphase 111 and 1112, rigid support 2 may be at a temperature substantially equal to a heating temperature, suitably substantially between 80°C and 130°C and, in detail, substantially equal to 120°C.

In construction phase 110, additive 36 may be added to at least part of OECT 3; said additive 36 being, in a preferred case, MIP made using solute as a mold molecule. Consequently, additive 36 may at least partially **stand** between OECT 3 and the arboreal fluid and, in detail, between channel 34 and/or 35 and the fluid by coming into contact with said fluid. Alternatively or additionally, additive 36 can at least partially stand between gate electrode 33 and second channel (35).

In detail, **construction phase** 110 may include a preparation subphase 113 of additive 36 preferably be MIP. Subphase of preparation 113 can therefore be a molecular imprinting procedure, a technique to create shaped cavities in polymeric matrices with predetermined selectivity, and high affinity to the solute. Additive 36 can thus be obtained using solute as a mold molecule (i.e. as a molecule to be used for the creation of MIP through a molecular imprinting technique that leaves cavities in the polymer matrix with an affinity for a chosen "mold" molecule).

In subphase 113 additive 36 can then be mixed with at least part of the electrically conductive material and then deposited on rigid support 2 creating channel 34 and/or 35. In detail, additive 36 can be mixed with the first electrically conductive material (and then deposited together with it in the first subphase) for the creation of channel 34 and/or 35. Alternatively or additionally, additive 36 can be mixed with at least part of the electrically conductive material used to make gate electrode 33.

As an alternative to mixing with electrically conductive material, additive 36 can define a coating of at least part of channel 34 and/or 35 and therefore said **construction phase** can include coating subphase **114** in which additive 36 can be deposited on at least part of channel 34 and/or 35 and/or gate electrode 33.

Consequently, additive 36 may at least partially stand between OECT 3 and the arboreal fluid and, in detail, between channel 34 and/or 35 and the fluid by coming into contact with said fluid. Alternatively or additionally, additive 36 can at least partially stand between gate electrode 33 and the second channel (35). Alternatively or additionally, it can at least partially stand between gate electrode 33 and the second channel (35).

Coating subphase 114 is subsequent to the first deposit subphase and preferably prior to the second deposit subphase.

In coating subphase 114, additive 36 may be applied to at least part of channel 34 and/or 35 as described above.

**Manufacturing process** 100 may comprise covering step **120** in which sheath 6 covers OECT 3 and at least part of rigid support 2. In particular, sheath 6 covers OECT 3 and active sector 2b.

**Process of making** 100 may comprise connection step **130** of board 4 to OECT 3 through at least one connector 5.

Optionally in connection step 140 power supply 7 can be connected to the rest of phyto-analysis sensor 1 through said board 4.

The operation of the phyto-analysis sensor 1 introduces a new procedure of detection **200** of a solute in a suitably arboreal fluid that can be implemented by sensor 1. Detection procedure 200 can be controlled from board 4.

Detection procedure 200 can be performed according to Fig. 8.

It may include insertion step **210** of support 2, OECT 3 (in detail, of at least channels 34 and/or 35) and, if present, of sheath 6 in stem 1a and, in detail, in hole 1c of plant 1b and, more in detail, of stem 1a substantially counter shaped to said rigid support 2. Detection procedure 200 can include wetting phase **220** in which support 2, being inserted into stem 1a (in detail, in contact with the conductive tissues of stem 1a) absorbs an arboreal fluid suitably arboreal passing from a dry to a wet condition. In detail, in phase 220 said fluid wets sheath 6 and through it comes into contact with rigid support 2 that absorbs it.

The fluid, through rigid support 2, comes into contact and wets the first channel (34) and if present, the second channel (35).

It should be noted that in the case of the first circumference-shaped channel (34), it is wet with the fluid regardless of the orientation of support 2 when inserted into stem 1a. Detection procedure 200 can include setting step **230** of sensor 1 in which the reference current of OECT 3 is determined, i.e. the current when not influenced by the solute.

In setting phase 230, the fluid absorbed by support 2 may contain at least said solute.

In setting phase 230 the reference current between drain electrode 31 and source electrode 32 can be determined by placing OECT 3 in the first acquisition configuration. The reference current measurement can be stored in the memory of board 4.

Detection procedure 200 may include at least one **measuring 240 phase** of the presence of solute in the fluid.

In measurement phase 240, the measuring current, i.e. the current in OECT 3 can be determined when influenced by the presence of solute in the fluid, appropriately between drain electrode 31 and source electrode 32.

In measurement phase 240 OECT 3 can be placed in the second acquisition configuration.

It should be noted that in said phase 240, thanks to additive 36, gate electrode 33 affects the passage of current in the first channel (34) between drain electrode 31 and source electrode 32 depending (appropriately and proportionally) on the presence of solute in the fluid absorbed by rigid support 2. In fact, the solute, if present in the fluid, interacts with additive 36 (in particular they are captured by it thanks to the molecular fingerprint) modifying the electrophysical characteristics of the first channel (34) and therefore the current between electrodes 31 and 32.

It should be noted that this action of the solute is determined by the fact that, if present in the fluid in contact with the molecular fingerprint and therefore with additive 36, the solute is interposed between the fluid and OECT 3 preventing any electrolytes/ions in the fluid from interacting with said OECT 3. This action can be amplified by the solute which, by defining a constraint on the electrostatic additive 36, varies the conductivity of OECT 3.

The content and thus the solute concentration are defined as a function of the difference between the measuring current and the reference current. In detail, a solute presence is detected if the difference between the measuring current and the reference current is at least equal to a detection threshold. Such detection threshold can be substantially equal to 1% of e.g. current reference.

Preferably, the solute content/concentration is directly proportional to the difference between the measuring current and the reference current.

Board 4 can determine this content/concentration of solute according to the parameter database and therefore to the characteristics of rigid support 2.

The measurement of the measuring current can be stored in the memory of board 4.

Once measurement phase 240 has been completed, detection procedure 200 may include phyto-analysis phase **250** in which a phyto-analysis of plant 1b is performed, evaluating the state of plant 1b according to said content/concentration of solute and the phyto-analysis database.

It should be noted that phyto-analysis phase 250 can be performed from board 4. Alternatively, board 4 can send, through these means of connection, the acquired data (content/concentration of solute) to an external unit which will carry out said phase of phyto analysis.

Detection procedure 200 may also include warning phase **260** in which board 4 sends an indication of the presence or absence of solute in the fluid, suitably arboreal, to the interface.

Phyto-analysis sensor 1, according to the invention, achieves important advantages.

In particular, compared to the well-known sensors, phyto-analysis sensor 1, thanks to the peculiar rigid support 2, is perfectly bio-compatible with plant 1b.

This aspect translates into a non-rejection of sensor 1 by plant 1b and therefore in the possibility of performing such analyzes for an extremely long time.

A non-secondary advantage in the simplicity of **construction** and installation/use of sensor 1 as highlighted in the above described embodiment 100 and detection procedure 200.

Another advantage lies in the fact that sensor 1, being able to detect the presence of a predefined solute , allows to perform, a precise real-time biochemical characterization of the fluid of plant 1b and then to concretely perform a phyto-analysis, for example detecting that the content of the solute is equal to that desired.

In particular, sensor 1 allows to carry out a precise continuous monitoring over time of the conditions of stem 1a and therefore of plant 1b. This advantage is given by the fact that rigid support 2, on which the OECT is made, is configured both to be inserted into stem 1a and to absorb the fluid from plant 1b. Consequently, having support 2 a structural function for OECT 3 and being a fluid absorber, sensor 1, unlike the well-known OECT sensors presenting "soft" absorbers such as paper or fabrics that would deform if inserted into a rigid body such as stem 1a, has a structure, defined by support 2 which isrigid and capable of absorbing and being penetrated by the fluids of stem 1a.

In conclusion, sensor 1 and, in detail, OECT 3 can be inserted in stem 1a without undergoing even small deformations that, being in an OECT the analysis strongly linked to the geometry of the same OECT, would lead to unpredictable changes in the absorption of the fluid and, above all, in the response of sensor 1 with consequent inability to perform a precise analysis of the arboreal fluid and an incorrect phyto-analysis of the plant.

A further advantage is given by the presence of a first channel (34) with closed projection profile. In fact, as demonstrated by tests performed by the inventor, the current trend in the first channel (34), having a first channel (34) surrounding the entire section of support 2, is immediately wet by the fluid allowing an immediate and greater variation of current during the second configuration and therefore a greater detection precision.

This ability to perform a better analysis is also given by the presence of sheath 6 which, absorbing the fluid, allows it to stagnate more in contact with rigid support 2, favoring its absorption by the same support 2.

A non-secondary advantage given by the presence of the sheath is that, overlapping between OECT 3 and being in electrically insulating material, it allows to avoid a passage of current between stem 1a and OECT 3 that could damage OECT 3 or plant 1b.

The invention is susceptible to variants within the scope of the inventive concept defined by the claims.

For example, OECT 3 may include an additional gate electrode sensitive to at least one additional solute in the tree fluid, preferably distinct from that detected by gate 33 electrode.

In a further example, which can be added to the previous one, phyto-analysis sensor 1, as shown in Fig. 2, may comprise two sensitive devices and, preferably, a first sensitive device comprising solute-specific additive 36 (e.g. glucose) and a second additive free sensitive device 36.

In this case, according to what is described above, in insertion phase 210 the two sensitive devices are inserted into two distinct holes 1c of plant 1b; in wetting phase 220 the supports 2 of the two sensitive devices absorb the arboreal fluid; in setting phase 230 the reference current of OECT 3 of the two sensitive devices is determined.

In measurement phase 240 the first sensing device determines the measuring current, i.e. the current in OECT 3 when influenced by the presence of the solute detected by additive 36 and ions (e.g. deriving from salts) present in the body fluid; while the second sensing device determines the measurement current, i.e. the current in OECT 3 when influenced only by ions present in the body fluid.

Therefore, in phyto-analysis phase 250 the presence of the solute is detected as a function of the current difference detected by the two sensitive devices.

In this context, all details can be replaced by equivalent elements and the materials, shapes and dimensions can be any.

## Claims

1. Phyto-analysis sensor (1) configured to be at least partially inserted into the stem (1a) of a plant (1b) and **characterized in that** it includes:
- a rigid support (2) designed to be inserted into said stem (1a) and to absorb at least one fluid of said plant (1b);
- an OECT (3) comprising
∘ a drain electrode (31);
∘ a source electrode (32);
∘ an electrically conductive first channel (34) connecting said drain electrode (31) to said source electrode (32) and integral to said rigid support (2) so as to be wet by said fluid absorbed by said rigid support (2);
∘ a gate electrode (33) sensitive to at least one solute in said fluid and configured to interact through said fluid absorbed by said rigid support (2) with said first channel (34) resulting in a variation of current in said first channel (34) and then between said drain electrode (31) and said source electrode (32), as a function of said solute **in that** fluid;
- a control board (4) configured to control said OECT (3) and define at least an acquisition configuration wherein a potential difference is defined between said drain electrode (31) and said source electrode (32); and
- at least one connector (5) from said board (4) to said OECT (3).

2. Phyto-analysis sensor
(1) according to claim 1, in which said OECT (3) includes an additive (36) which is electrically sensitive to said solute and placed between that first channel (34) and said rigid support (2); and in which said additive (36) being MIP made using solute as a mold molecule.

3. Phyto-analysis sensor
(1) according to at least one previous claim, including a board (4) configured to determine the content of said solute as a function of said current change in said first channel (34) and then between said drain electrode (31) and said source electrode (32).

4. Phyto-analysis sensor
(1) according to at least one previous claim, in which said rigid support (2) is made of a material having a Brinell hardness substantially between 25 N/mm 2 and 50 N/mm 2 and a porosity substantially between 50% and 75%.

5. Phyto-analysis sensor (1) according to claim 4, in which said rigid support (2) is made of bamboo or rattan.

6. Phyto-analysis sensor
(1) according to at least one previous claim, in which said first channel (34) defines a closed projection profile in a plane perpendicular to a longitudinal axis (2a) and passing through said drain electrode (31) and said source electrode (32).

7. Phyto-analysis sensor
(1) according to at least one previous claim, including a sheath (6) covering at least part of saidrigid support (2) enclosing said electrodes (31, 32, 33) and said first channel (34) between said rigid support (2) and said sheath (4); said sheath (4) is made of an electrically insulating and hydrophilic material which can absorb said fluid of said plant (1b) and thus be penetrated by it .

8. Phyto-analysis sensor
(1) according to at least one previous claim, in which said gate electrode (33) and source electrode (32) are placed at a first section of said rigid support (2); in which said gate electrode (33) is placed at a second section of said rigid support(2) distinct from said first section.

9. Phyto-analysis sensor
(1) according to the above claim, in which the distance between said first section and said second section is substantially between 1 mm and 3 mm.

10. Phyto-analysis sensor (1) according to at least one of claims 8-9, in which the distance
between said first section and said second section is substantially equal to said distance between said gate electrode (33) and said source electrode (32).

11. Phyto-analysis sensor
(1) according to at least one of claims 8-10, in which said first channel (34) develops along the entire perimeter of said first section.

12. Procedure for the construction (100) of a phyto-analysis sensor (1) according to claim 1, configured to be at least partially inserted into a stem (1a) of a plant (1b) **characterized in that** said phyto-analysis sensor (1) includes:
- a rigid support (2) designed to be inserted into said stem (1a) and to absorb at least one fluid of said plant (1b);
- an OECT (3) comprising a drain electrode (31; a source electrode (32); an electrically conductive first channel (34) connecting the drain electrode (31) to the source electrode (32); and a gate electrode (33) sensitive to at least one solute **in that** fluid; and by the fact that this construction procedure (100) includes:
- a construction phase (110) in which said OECT (3) is deposited on said rigid support (2) so that said first channel (34) and said gate electrode (33) are wetted by said fluid absorbed by said rigid support (2) allowing said gate electrode (33) to interact through said rigid support (2) with said first channel (34) causing a variation of current in said first channel (34) and therefore between said drain electrode (31) and said source electrode (32) as a function of said solute **in that** fluid.

13. Construction procedure (100) according to the previous claim, in which at said construction phase (110) said OECT (3) is deposited on said rigid support (2) by adding an additive (36) electrically sensitive to said solute to at least part of that circuit (3); said additive (36) MIP being made using the mould molecule called solute.

14. Detection procedure (200) of a solute in a plant fluid (1b) using a phyto-analysis sensor (1) according to claim 1, **characterized in that** said phyto-analysis sensor (1) comprises:
- a rigid support (2) designed to be inserted into said stem (1a) and to absorb at least one fluid of said plant (1b);
- an OECT (3) comprising
∘ a drain electrode (31);
∘ a source electrode (32); an electrically conductive first channel (34) connecting said drain electrode (31) to said source electrode (32) and integral to said rigid support (2);
∘ a gate electrode (33) sensitive to at least one solute in said fluid and configured to interact through said rigid support (2) with said first channel (34) causing a variation of current in said first channel (34) and thus between said drain electrode (31) and said source electrode (32) as a function of said solute **in that** fluid;
said detection procedure also includes:
- a setting phase (230) of said sensor (1) in which the reference current of said OECT (3) is determined and not affected by said solute in said fluid;
- a measurement phase (240) of the presence of this solute in this fluid;
wherein during said measurement phase the following is determined: the measuring current of said OECT (3) affected by said solute in the fluid; the presence of said solute by comparing said reference current with said measured current.

15. Detection procedure (200) according to the previous claim, comprising a phyto-analysis database associating the value of a physiological parameter of said plant (1b) with said content of said solute; and a phyto-analysis phase (250) in which a phyto-analysis of said plant (1b) is performed by evaluating the state of plant according to said solute content and said phyto-analysis database.

## Patentansprüche

1. Phytoanalyse-Sensor (1), der so konfiguriert ist, dass er zumindest teilweise in den Stiel (1a) einer Pflanze (1b) eingeführt werden kann, und **dadurch gekennzeichnet ist, dass** er Folgendes umfasst:
- einen starren Träger (2), der dazu bestimmt ist, in den Stamm (1a) eingeführt zu werden und mindestens eine Flüssigkeit der Pflanze (1b) aufzunehmen;
- einen OECT (3) mit:
∘ eine Drainageelektrode (31);
∘ eine Source-Elektrode (32)
∘ einen elektrisch leitfähigen ersten Kanal (34), der die Drain-Elektrode (31) mit der Source-Elektrode (32) verbindet und integraler Bestandteil des starren Trägers (2) ist, so dass er von der vom starren Träger (2) absorbierten Flüssigkeit benetzt wird.
∘ eine Gate-Elektrode (33), die auf mindestens einen gelösten Stoff in der Flüssigkeit anspricht und so konfiguriert ist, dass sie über die von dem starren Träger (2) absorbierte Flüssigkeit mit dem ersten Kanal (34) interagiert, was zu einer Stromänderung in dem ersten Kanal (34) und dann zwischen der Drain-Elektrode (31) und der Source-Elektrode (32) in Abhängigkeit von dem gelösten Stoff in dieser Flüssigkeit führt;
- eine Steuerplatine (4), die so konfiguriert ist, dass sie das OECT (3) steuert und mindestens eine Erfassungskonfiguration definiert, in der eine Potentialdifferenz zwischen der Drain-Elektrode (31) und der Source-Elektrode (32) definiert ist; und mindestens einen Verbinder (5) von der Platine (4) zum OECT (3).

2. Phytoanalysesensor (1) gemäß Anspruch 1, wobei das OECT (3) einen Zusatzstoff (36) enthält, der elektrisch empfindlich auf den gelösten Stoff reagiert und zwischen dem ersten Kanal (34) und der starren Halterung (2) angeordnet ist; und wobei der Zusatzstoff us (36) ein MIP ist, das unter Verwendung des gelösten Stoffes als Formmolekül hergestellt wurde.

3. Phytoanalysesensor (1) gemäß mindestens einem vorherigen Anspruch, der eine Platine (4) umfasst, die so konfiguriert ist, dass sie den Gehalt des gelösten Stoffes als Funktion der Stromänderung in dem ersten Kanal (34) und dann zwischen der Drain-Elektrode (31) und der Source-Elektrode (32) bestimmt.

4. Phytoanalysesensor (1) gemäß mindestens einem der vorhergehenden Ansprüche, wobei die starre Halterung (2) aus einem Material mit einer Brinell-Härte zwischen 25 N/mm² und 50 N/mm² und einer Porosität zwischen 50 % und 75 % hergestellt ist.

5. Phytoanalysesensor (1) gemäß Anspruch 4, wobei der starre Träger (2) aus Bambus oder Rattan besteht.

6. Phytoanalyse-Sensor (1) gemäß mindestens einem vorherigen Anspruch, wobei der erste Kanal (34) ein geschlossenes Projektionsprofil in einer Ebene definiert, die senkrecht zu einer Längsachse (2a) steht und durch die Drain-Elektrode (31) und die Source-Elektrode (32) verläuft.

7. Phytoanalysesensor (1) gemäß mindestens einem der vorhergehenden Ansprüche, mit einer Hülle (6), die mindestens einen Teil des starren Trägers (2) bedeckt und die Elektroden (31, 32, 33) und den ersten Kanal (34) zwischen dem starren Träger (2) und der Hülle (4) umschließt; wobei die Hülle (4) aus einem elektrisch isolierenden und hydrophilen Material besteht, das die Flüssigkeit der Pflanze (1b) absorbieren kann und somit von dieser durchdrungen werden kann.

8. Phytoanalysesensor (1) gemäß mindestens einem der vorhergehenden Ansprüche, wobei die Gate-Elektrode (33) und die Source-Elektrode (32) an einem ersten Abschnitt des starren Trägers (2) angeordnet sind; wobei die Gate-Elektrode (33) an einem zweiten Abschnitt des starren Trägers (2) angeordnet ist, der sich von dem ersten Abschnitt unterscheidet.

9. Phytoanalysesensor (1) gemäß dem vorstehenden Anspruch, wobei der Abstand zwischen dem ersten Abschnitt und dem zweiten Abschnitt im Wesentlichen zwischen 1 mm und 3 mm beträgt.

10. Phytoanalysesensor gemäß mindestens einem der Ansprüche 8-9, wobei der Abstand zwischen dem ersten Abschnitt und dem zweiten Abschnitt im Wesentlichen gleich dem Abstand zwischen der Gate-Elektrode (33) und der Source-Elektrode (32) ist.

11. Phytoanalysesensor gemäß mindestens einem der Ansprüche 8-10, wobei sich der erste Kanal (34) entlang des gesamten Umfangs des ersten Abschnitts erstreckt.

12. Verfahren zur Herstellung (100) eines Phytoanalysesensors (1) gemäß Anspruch 1, der so konfiguriert ist, dass er zumindest teilweise in einen Stiel (1a) einer Pflanze (1b) eingeführt werden kann, **dadurch gekennzeichnet, dass** der Phytoanalysesensor (1) umfasst:
- einen starren Träger (2), der dazu bestimmt ist, in den Stamm (1a) eingeführt zu werden und mindestens eine Flüssigkeit der Pflanze (1b) aufzunehmen;
- einen OECT (3), der umfasst:
∘ eine Drainageelektrode (31);
∘ eine Source-Elektrode (32);
∘ einen elektrisch leitfähigen ersten Kanal (34), der die Drainage - Elektrode (31) mit der Source-Elektrode (32) verbindet;
∘ eine Gate-Elektrode (33), die auf mindestens einen gelösten Stoff in diesem Fluid anspricht;
- und dadurch, dass dieses Konstruktionsverfahren (100) umfasst:
∘ eine Konstruktionsphase (110), in der das OECT (3) auf dem starren Träger (2) abgeschieden wird, so dass der erste Kanal (34) und die Gate-Elektrode (33) durch die vom starren Träger (2) absorbierte Flüssigkeit benetzt werden, wodurch die Gate-Elektrode (33) über den starren Träger (2) mit dem ersten Kanal (34) interagieren kann, was zu einer Stromänderung im ersten Kanal (34) und somit zwischen der Drain-Elektrode (31) und der Source-Elektrode (32) in Abhängigkeit von dem in diesem Fluid gelösten Stoff bewirkt wird.

13. Herstellungsverfahren (100) gemäß dem vorherigen Anspruch, bei dem in der Herstellungsphase (110) der OECT (3) auf dem starren Träger (2) abgeschieden wird, indem ein Additiv (36), das elektrisch empfindlich auf den gelösten Stoff reagiert, zu mindestens einem Teil dieser Schaltung (3) hinzugefügt wird; wobei das Additiv (36) MIP unter Verwendung des als gelöster Stoff bezeichneten Formmoleküls hergestellt wird.

14. Verfahren (200) zum Nachweis eines gelösten Stoffes in einer Pflanzenflüssigkeit (1b) unter Verwendung eines Phytoanalysesensors (1) gemäß Anspruch 1, **dadurch gekennzeichnet, dass** der Phytoanalysesensor (1) umfasst:
- einen starren Träger (2), der dazu bestimmt ist, in den Stamm (1a) eingeführt zu werden und mindestens eine Flüssigkeit der Pflanze (1b) aufzunehmen;
- ein OECT (3), das umfasst:
∘ eine Drain-Elektrode (31);
∘ eine Source-Elektrode (32); einen elektrisch leitfähigen ersten Kanal (34) , der die Drain-Elektrode (31) mit der Source-Elektrode (32) verbindet und integraler Bestandteil des starren Trägers (2) ist;
∘ eine Gate-Elektrode (33), die auf mindestens einen gelösten Stoff in der Flüssigkeit anspricht und so konfiguriert ist, dass sie über die starre Stütze (2) mit dem ersten Kanal (34) interagiert und eine Stromänderung im ersten Kanal (34) und damit zwischen der Drain-Elektrode (31) und der Source-Elektrode (32) in Abhängigkeit von dem gelösten Stoff in dieser Flüssigkeit bewirkt;
- das Detektionsverfahren umfasst außerdem:
∘ eine Einstellphase (230) des Sensors (1), in der der Referenzstrom des OECT (3) bestimmt wird und nicht durch den gelösten Stoff in der Flüssigkeit beeinflusst wird.
∘ eine Messphase (240) des Vorhandenseins dieses gelösten Stoffes in dieser Flüssigkeit; wobei während der Messphase Folgendes bestimmt wird:
▪ der Messstrom des OECT (3), der durch den gelösten Stoff in der Flüssigkeit beeinflusst wird.
▪ das Vorhandensein des gelösten Stoffes durch Vergleichen des Referenzstroms mit dem gemessenen Strom.

15. Erfassungsverfahren (200) gemäß dem vorherigen Anspruch, umfassend eine Phytoanalyse-Datenbank, die den Wert eines physiologischen Parameters der Pflanze (1b) mit dem Gehalt des gelösten Stoffes in Verbindung bringt; und eine Phytoanalysephase (250), in der eine Phytoanalyse der Pflanze (1b) durchgeführt wird, indem der Zustand der Pflanze gemäß dem Gehalt des gelösten Stoffes und der Phytoanalyse-Datenbank bewertet wird.

## Revendications

1. Capteur de phyto-analyse (1) configuré pour être au moins partiellement inséré dans la tige (1a) d'une plante (1b) et **caractérisé en ce qu'**il comprend :
- un support rigide (2) conçu pour être inséré dans ladite tige (1a) et pour absorber au moins un fluide de ladite plante (1b) ;
- un OECT (3) comprenant :
∘ une électrode de drain (31) ;
∘ une électrode de source (32)
∘ un premier canal électriquement conducteur (34) reliant ladite électrode de drain (31) à ladite électrode de source (32) et solidaire dudit support rigide (2) de manière à être mouillé par ledit fluide absorbé par ledit support rigide (2).
∘ une électrode de grille (33) sensible à au moins un soluté dans ledit fluide et configurée pour interagir à travers ledit fluide absorbé par ledit support rigide (2) avec ledit premier canal (34), entraînant une variation de courant dans ledit premier canal (34), puis entre ladite électrode de drain (31) et ladite électrode de source (32), en fonction dudit soluté dans ce fluide ;
- une carte de commande (4) configurée pour commander ledit OECT (3) et définir au moins une configuration d'acquisition dans laquelle une différence de potentiel est définie entre ladite électrode de drain (31) et ladite électrode de source (32) ; et au moins un connecteur (5) reliant ladite carte (4) audit OECT (3).

2. Capteur de phyto-analyse (1) selon la revendication 1, dans lequel ledit OECT (3) comprend un additif (36) qui est électriquement sensible audit soluté et placé entre ledit premier canal (34) et ledit support rigide (2) ; et dans lequel ledit additif (36) est un MIP fabriqué à l'aide du soluté comme molécule de moulage.

3. Capteur de phytoanalyse (1) selon au moins une revendication précédente, comprenant une carte (4) configurée pour déterminer la teneur en ledit soluté en fonction de ladite variation de courant dans ledit premier canal (34), puis entre ladite électrode de drain (31) et ladite électrode de source (32).

4. Capteur de phytoanalyse (1) selon au moins une revendication précédente, dans lequel ledit support rigide (2) est constitué d'un matériau ayant une dureté Brinell comprise entre 25 N/mm² et 50 N/mm² et une porosité comprise entre 50 % et 75 %.

5. Capteur de phytoanalyse (1) selon la revendication 4, dans lequel ledit support rigide (2) est constitué de bambou ou de rotin.

6. Capteur de phytoanalyse (1) selon au moins une revendication précédente, dans lequel ledit premier canal (34) définit un profil en saillie fermé dans un plan perpendiculaire à un axe longitudinal (2a) et passant par ladite électrode de drain (31) et ladite électrode de source (32).

7. Capteur de phyto-analyse (1) selon au moins une revendication précédente, comprenant une gaine (6) recouvrant au moins une partie dudit support rigide (2) renfermant lesdites électrodes (31, 32, 33) et ledit premier canal (34) entre ledit support rigide (2) et ladite gaine (4) ; ladite gaine (4) étant réalisée en un matériau électriquement isolant et hydrophile, susceptible d'absorber ledit fluide de ladite plante (1b) et donc d'être pénétrée par celui-ci.

8. Capteur de phytoanalyse (1) selon au moins une revendication précédente, dans lequel ladite électrode de grille (33) et ladite électrode de source (32) sont placées au niveau d'une première section dudit support rigide (2) ; dans lequel ladite électrode de grille (33) est placée au niveau d'une deuxième section dudit support rigide (2) distincte de ladite première section.

9. Capteur de phytoanalyse (1) selon la revendication ci-dessus, dans lequel la distance entre ladite première section et ladite deuxième section est sensiblement comprise entre 1 mm et 3 mm.

10. Capteur de phytoanalyse selon au moins l'une des revendications 8 à 9, dans lequel la distance entre ladite première section et ladite deuxième section est sensiblement égale à ladite distance entre ladite électrode de grille (33) et ladite électrode de source (32).

11. Capteur de phytoanalyse selon au moins l'une des revendications 8 à 10, dans lequel ledit premier canal (34) s'étend sur tout le périmètre de ladite première section.

12. Procédé de fabrication (100) d'un capteur de phytoanalyse (1) selon la revendication 1, configuré pour être au moins partiellement inséré dans une tige (1a) d'une plante (1b), **caractérisé en ce que** ledit capteur de phytoanalyse (1) comprend :
- un support rigide (2) conçu pour être inséré dans ladite tige (1a) et pour absorber au moins un fluide de ladite plante (1b) ;
- un OECT (3) comprenant :
∘ une électrode de drain (31) ;
∘ une électrode de source (32) ;
∘ un premier canal électriquement conducteur (34) reliant l'électrode de drain (31) à l'électrode de source (32) ;
∘ une électrode de grille (33) sensible à au moins un soluté dans ce fluide ;
- et par le fait que cette procédure de construction (100) comprend :
∘ une phase de construction (110) dans laquelle ledit OECT (3) est déposé sur ledit support rigide (2) de telle sorte que ledit premier canal (34) et ladite électrode de grille (33) soient mouillés par ledit fluide absorbé par ledit support rigide (2), permettant à ladite électrode de grille (33) d'interagir à travers ledit support rigide (2) avec ledit premier canal (34), provoquant une variation de courant dans ledit premier canal (34) et donc entre ladite électrode de drain (31) et ladite électrode de source (32) en fonction dudit soluté dans ce fluide.

13. Procédé de construction (100) selon la revendication précédente, dans lequel, lors de ladite phase de construction (110), ledit OECT (3) est déposé sur ledit support rigide (2) en ajoutant un additif (36) électriquement sensible audit soluté à au moins une partie de ce circuit (3) ; ledit additif (36) MIP étant fabriqué à l'aide de la molécule moule appelée soluté.

14. Procédé de détection (200) d'un soluté dans un fluide végétal (1b) à l'aide d'un capteur de phyto-analyse (1) selon la revendication 1, **caractérisé en ce que** ledit capteur de phyto-analyse (1) comprend :
- un support rigide (2) conçu pour être inséré dans ladite tige (1a) et pour absorber au moins un fluide de ladite plante (1b) ;
- un OECT (3) comprenant :
∘ une électrode de drain (31) ;
∘ une électrode de source (32) ; un premier canal électriquement conducteur (34) reliant ladite électrode de drain (31) à ladite électrode de source (32) et solidaire dudit support rigide (2) ;
∘ une électrode de grille (33) sensible à au moins un soluté dans ledit fluide et configurée pour interagir à travers ledit support rigide (2) avec ledit premier canal (34), provoquant une variation de courant dans ledit premier canal (34) et donc entre ladite électrode de drain (31) et ladite électrode de source (32) en fonction dudit soluté dans ce fluide;
- ladite procédure de détection comprend également :
∘ une phase de réglage (230) dudit capteur (1) dans laquelle le courant de référence dudit OECT (3) est déterminé et n'est pas affecté par ledit soluté dans ledit fluide.
∘ une phase de mesure (240) de la présence de ce soluté dans ce fluide ; dans laquelle, pendant ladite phase de mesure, on détermine :
▪ le courant de mesure dudit OECT (3) affecté par ledit soluté dans le fluide.
▪ la présence dudit soluté en comparant ledit courant de référence avec ledit courant mesuré.

15. Procédé de détection (200) selon la revendication précédente, comprenant une base de données de phyto-analyse associant la valeur d'un paramètre physiologique de ladite plante (1b) à ladite teneur en ledit soluté ; et une phase de phyto-analyse (250) dans laquelle une phyto-analyse de ladite plante (1b) est effectuée en évaluant l'état de la plante en fonction de ladite teneur en soluté et de ladite base de données de phyto-analyse.
